(19) Europäisches Patentamt
European Patent Office
Office européen des brevets

(11) Publication number: **0 415 375 A2**

# (12) EUROPEAN PATENT APPLICATION

(21) Application number: **90116516.7**

(22) Date of filing: **28.08.90**

(51) Int. Cl.5: **G03C 7/36**, //C07D233/90, C07D233/66

(30) Priority: **29.08.89 JP 223474/89**

(43) Date of publication of application: **06.03.91 Bulletin 91/10**

(84) Designated Contracting States: **DE GB**

(71) Applicant: **KONICA CORPORATION**
**26-2, Nishishinjuku 1-chome, Shinjuku-ku**
**Tokyo 160(JP)**

(72) Inventor: **Kida, Shuji, Konica Corporation**
**1 Sakura-machi**
**Hino-shi, Tokyo(JP)**
Inventor: **Sugita, Shuichi, Konica Corporation**
**1 Sakura-machi**
**Hino-shi, Tokyo(JP)**
Inventor: **Mizukura, Noboru, Konica Corporation**
**1 Sakura-machi**
**Hino-shi, Tokyo(JP)**
Inventor: **Tomotake, Atsushi, Konica Corporation**
**1 Sakura-machi**
**Hino-shi, Tokyo(JP)**

(74) Representative: **Henkel, Feiler, Hänzel & Partner**
**Möhlstrasse 37**
**W-8000 München 80(DE)**

## (54) Silver halide colour photographic light-sensitive material.

(57) A silver halide color photographic light-sensitive material using therein an active site substitution type yellow-dye forming coupler excellent in dispersion stability and rectivity is disclosed. The color photographic light-sensitive material comprises a layer containing a coupler represented by the following formula :

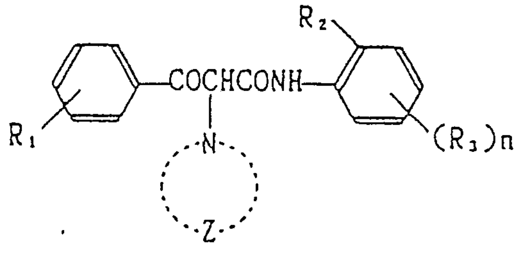

wherein at least one of $R_1$, $R_2$ and $R_3$ is a branched alkyl group having not less than 4 carbon atoms containing an asymmetric carbon atom, or a group containing said branched alkyl group.

EP 0 415 375 A2

# SILVER HALIDE COLOR PHOTOGRAPHIC LIGHT-SENSITIVE MATERIAL

## FIELD OF THE INVENTION

This invention relates to a silver halide color photographic light-sensitive material using therein an active site substitution type yellow-dye forming coupler excellent in color developability, image quality and dispersion stability.

## BACKGROUND OF THE INVENTION

In color photography as generally known, an aromatic primary amine type color develping agent oxidized by color developing a silver halide color photographic light-sensitive material and a coupler are coupled to each other, so that such a dye as an indophenone, indoaniline or azomethine dye may be produced and thereby forming a color image.

From the aspects of the recent strong intentions of making sensitivities and image qualities higher and making processing time shorter, highly reactive couplers have been demanded.

To be more detailed, the sensitivity of a light-sensitive material can be improved by increasing the reactivity of a coupler with the oxidized product of a developing agent. If it is not particularly required to make a sensitivity higher, as much couplers and silver halides can be reduced by increasing the reactivity of the couplers. Resultingly, the layer thickness of the light-sensitive material can be so thinned as to reduce the scattering of incident light, therefore, the sharpness can be improved. In color-negative and color-reversal light-sensitive materials, the best effect can usually be displayed by thinning the thickness of the blue light-sensitive layer thereof, because the blue light-sensitive layer is the closest to the side of incident light. Accordingly, the developments of highly reactive yellow couplers have particularly been demanded.

Such yellow couplers include, typically, a pivaloyl acetoanilide type yellow coupler and a benzoyl acetoanilide type yellow coupler.

It has generally been known that the benzoyl acetoanilide type yellow couplers are higher in reactivity than others. However, the conventional benzoyl acetoanilide type couplers have a defect in that the solubility to a high boiling organic solvent is substantially low.

It is, therefore, difficult to use any highly reactive benzoyl acetoanilide type coupler independently, so that a low reactive pivaloyl acetoanilide type coupler has sometimes been compelled to use in combination so as to prevent undesired deposition. Therefore, it has been unable to make the layer thinner and to expect any satisfactory effect of sharpness improvement.

For counterchecking the above disadvantages, European Patent No. 327,348 proposes for a benzoylacetoanilide type coupler in which the solubility is improved by introducing a branched alkyl group having an optically active carbon thereinto. This type couplers have the defect in that the color developability is deteriorated when using a developer having a low pH, though the couplers apparently improve the solubility thereof.

## SUMMARY OF THE INVENTION

Taking the above-described problems remaining heretofore unsolved into consideration, it is, accordingly, an object of the invention to provide a silver halide color photographic light-sensitive material which is excellent in color developability, satisfactory in a maximum color density and high in sensitivity and image quality, when containing therein a yellow coupler having a satisfactory dispersion stability to a silver halide emulsion and a high reactivity.

The above-described objects of the invention can be achieved with a silver halide color photographic light-sensitive material comprising at least one of the layers each containing the couplers represented by the following Formula I:

Formula I

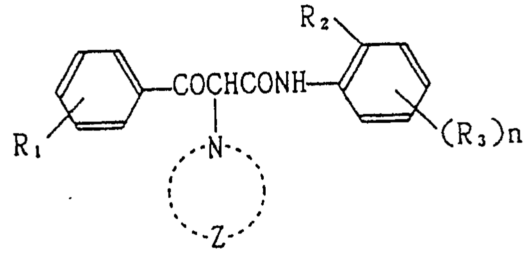

wherein $R_1$ represents a hydrogen atom, an alkyl group, an alkoxy group or an acylamino group; $R_2$ represents a halogen atom, a trifluoromethyl group or an alkoxy group; $R_3$ represents an L-$R_4$ or a halogen atom, in which L represents -OCO-, -COO-, -O-, -S-, -$SO_2$-,

```
-NSO2-,  -SO2N-,   -NCO-,  -CON-,
 |             |    |        |
 R5            R5   R5       R5
```

or -$OSO_2$-, $R_4$ represents an alkyl group or an aryl group, and $R_5$ represents a hydrogen atom or an alkyl group; n is an integer of 0, 1 or 2; and Z represents a group consisting of non-metal atoms necessary for producing a 5 ~ 6 membered heterocyclic ring; provided, when n is 2, $R_3$s may be the same with or different from each other. At least one of $R_1$, $R_2$ and n numbers of $R_3$ is a branched alkyl group having not less than 4 carbon atoms containing an asymmetric carbon atom, or a group containing the branched alkyl group.

The couplers of the invention will further be detailed below.

$R_1$ denoted in Formula I includes, preferably, a hydrogen atom, a straight-chained or branched alkyl group having 1 ~ 30 carbon atoms, a straight-chained or branched alkoxy group having 1 ~ 30 carbon atoms, and a straight-chained or branched alkylcarbonylamino or arylcarbonylamino group having 2 ~ 30 carbon atoms. Each of the groups represented by $R_1$ includes those having each substituents. The substituents include, for example, a halogen atom, an aryl group, an aryloxy group, an alkylthio group, an alkylsulfonyl group, an arylsulfonyl group, an acylamino group, an alkoxy group, and a hydroxyl group. Among the $R_1$, an alkoxy group is most preferable.

$R_2$ denoted in Formula I includes, preferably, a halogen atom, a trifluoromethyl group and a straight-chained or branched alkoxy group having 1 ~ 30 carbon atoms. The alkoxy groups include, further, those having each substituents. The substituents include, for example, those exemplified as the substituents to each of the groups represented by $R_1$. The particularly preferable $R_2$ is a chlorine atom.

In Formula I, $R_4$ represents, preferably, a straight-chained or branched alkyl group having 1 ~ 30 carbon atoms, and an aryl group such as a phenyl group and a naphthyl group. Each of the groups represented by $R_4$ includes those having substituents. Such substituents include, for example, those exemplified as the substituents to each of the groups represented by $R_1$. $R_4$ represents an alkyl group as the particularly preferable.

In Formula I, L represents, preferably, -COO-,

```
-NSO2-,
 |
 R5
```

```
-SO2N-
     |
     R5
```

or -$OSO_2$- and among them, -COO- is most preferable.

In Formula I, $R_5$ represents, preferably, a hydrogen atom or a straight-chained or branched alkyl group having 1 ~ 30 carbon atoms.

The groups each represented by $R_5$ include those having substituents. Such substituents include, for

example, those exemplified as the substituents to each of the groups represented by $R_1$.

In Formula I, the 5- ~ 6-membered heterocyclic rings produced by Z include, preferably, those represented by the following Formulas II ~ VII.

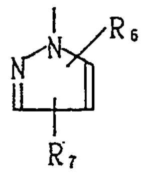

II

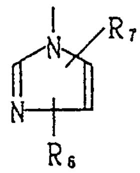

III

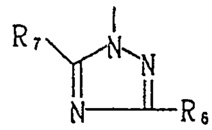

IV

wherein $R_6$ and $R_7$ each represent a hydrogen atom, a halogen atom, an alkyl group, an alkoxy group, an aryl group, a heterocyclic group, an alkoxycarbonyl group, an aryloxycarbonyl group, an amino group, an acylamino group, an alkylsulfonyl group, an arylsulfonyl group, an alkylsulfinyl group, an arylsulfinyl group, an alkylsulfonamido group, an arylsulfonamido group or a carboxyl group, and they include those having substituents. $R_6$ and $R_7$ are also allowed to form a ring.

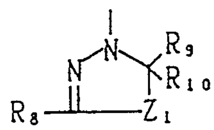

V

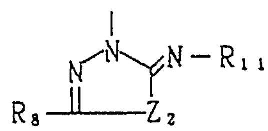

VI

wherein $Z_1$ and $Z_2$ represent each a hetero atom such as an oxygen atom; and $R_8$, $R_9$ and $R_{10}$ represent each the same groups as those represented by the foregoing $R_1$ and $R_7$; and $R_{11}$ represents an alkyl group, an aryl group, an alkylcarbonyl group, an arylcarbonyl group, an alkylsulfonyl group and an arylsulfonyl group, and they include those having substituents.

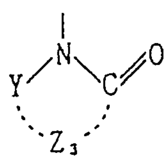

VII

wherein Y represents a hetero atom such as -N=, -NH-, -O-and -S-, a sulfonyl group, a carbonyl group, or a carbon atom represented by

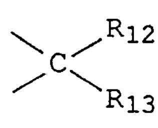

or

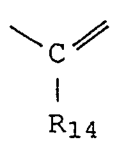

;

and $Z_3$ represents a group consisting of non-metal atoms necessary for producing a 5- ~ 6- membered ring in association with

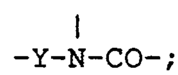

and

$R_{12}$, $R_{13}$ and $R_{14}$ represent the same groups as those represented by the foregoing $R_6$ and $R_7$, respectively, and $R_{12}$, $R_{13}$ and $R_{14}$ are also allowed to produce a ring, in association with a part of the rings produced by $Z_3$.

The 2-equivalent yellow coupler represented by the foregoing Formula I are also allowed to produce a bis-derivative upon coupling to $R_1$, $R_2$ and $R_3$.

At least one group of $R_1$, $R_2$ and n numbers of $R_3$ is a branched alkyl group having not less than 4 carbon atoms containing an asymmetric carbon atom or a group containing the branched alkyl group. Such branched alkyl groups include those having substituents. The preferable groups include, for example, the following groups:

$-CHCH_2CH_3$ | $CH_3$ ,
$-CH(CH_2)_2CH_3$ | $CH_3$ ,
$-CH(CH_2)_3CH_3$ | $CH_3$ ,

$-CH(CH_2)_4CH_3$ | $CH_3$ ,
$-CH(CH_2)_5CH_3$ | $CH_3$ ,
$-CH(CH_2)_9CH_3$ | $CH_3$ ,

$-CH(CH_2)_{11}CH_3$ | $CH_3$ ,
$-CH(CH_2)_{13}CH_3$ | $CH_3$ ,
$-CH(CH_2)_2CH_3$ | $C_2H_5$ ,

$-CH(CH_2)_3CH_3$ | $C_2H_5$ ,
$-CH(CH_2)_4CH_3$ | $C_2H_5$ ,
$-CH(CH_2)_8CH_3$ | $C_2H_5$ ,

$-CH(CH_2)_{10}CH_3$ | $C_2H_5$ ,
$-CH(CH_2)_5CH_3$ | $(CH_2)_4CH_3$ ,
$-CH_2CH(CH_2)_7CH_3$ | $(CH_2)_5CH_3$ ,

$-CH_2CHCH_2CH(CH_2)_2CH_3$ | | $C_2H_5$ $C_2H_5$ ,
$-CH_2CH_2OCHCH_2CH_3$ | $CH_3$ ,

$-CH_2CH_2SCHCH_2CH_3$ | $CH_3$ ,
$-CH_2CH_2SO_2CHCH_2CH_3$ | $CH_3$ ,

$-CH_2CH_2OCOCHCH_2CH_3$ | $CH_3$ ,
$-CH_2COOCH(CH_2)_2CH_3$ | $CH_3$ ,

$-CH_2CONHCH_2CH_2OCHCH_2CH_3$ | $CH_3$ ,
$-CHCH_2OCH_3$ | $CH_3$

$-CH(CH_2)_{11}CH_3$ | $CH_2COOCH_3$ .

Next, the typical examples of the yellow couplers of the invention will be given below.

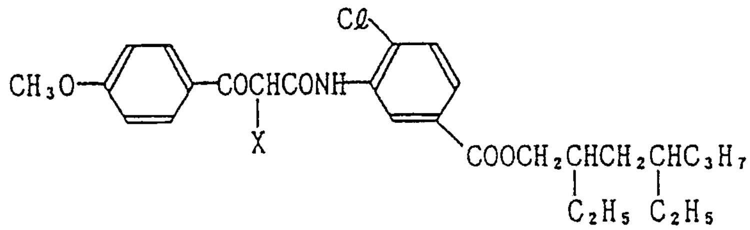

| No. | X | No. | X |
|---|---|---|---|
| (1) | 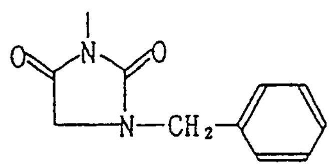 | (5) | 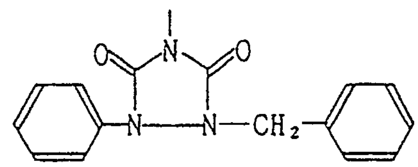 |
| (2) | 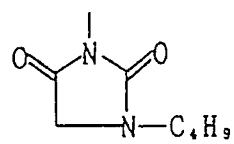 | (6) | 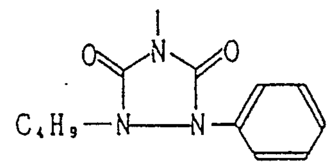 |
| (3) | 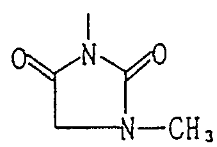 | (7) | 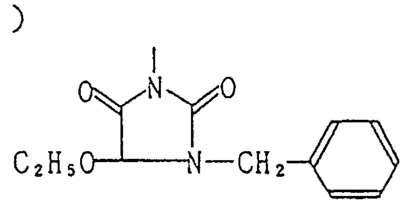 |
| (4) | 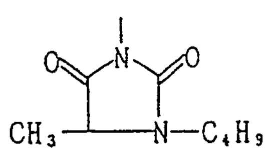 | (8) | 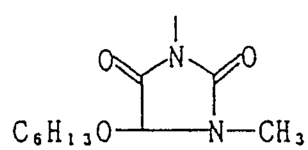 |

(9)

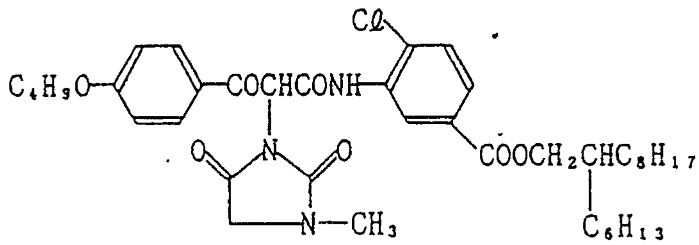

(10)

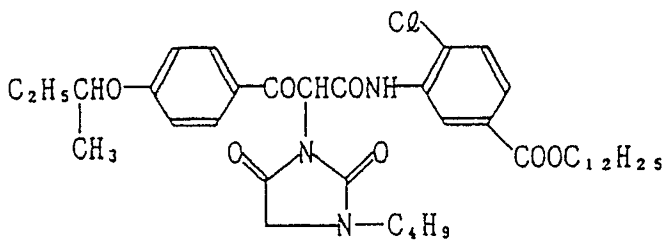

(11)

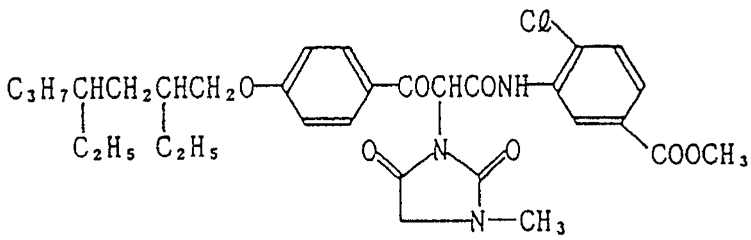

(12)

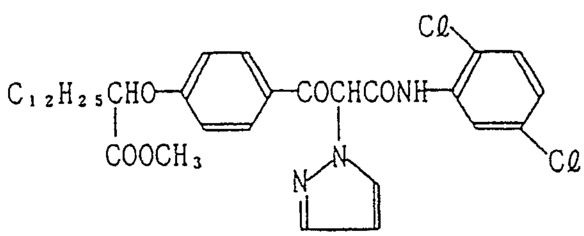

(13)

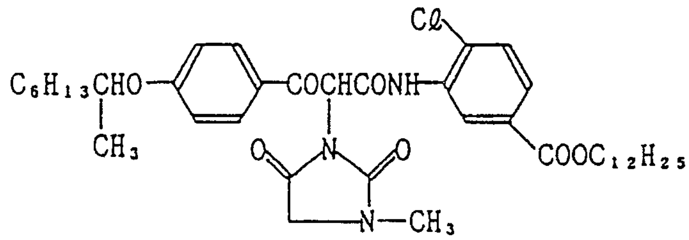

(14)

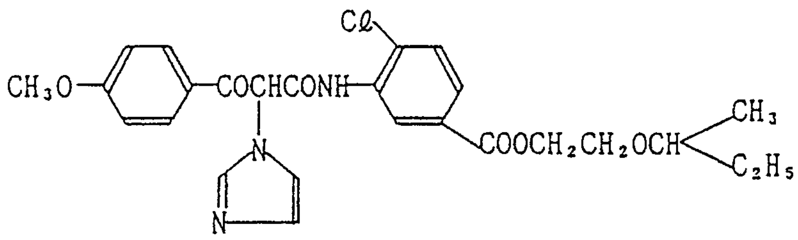

(15)

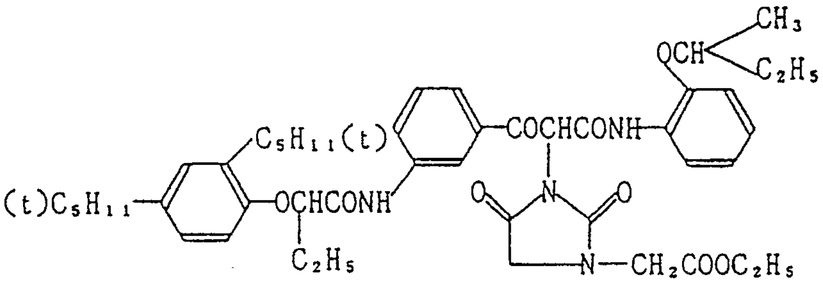

(16)

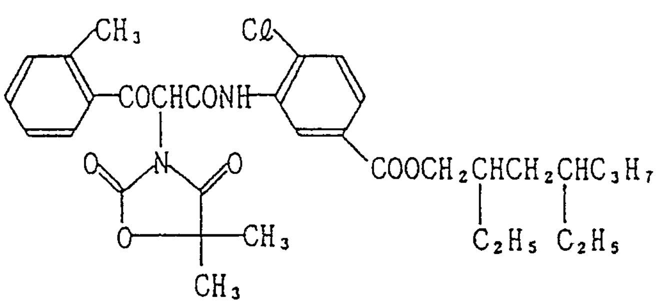

(17)

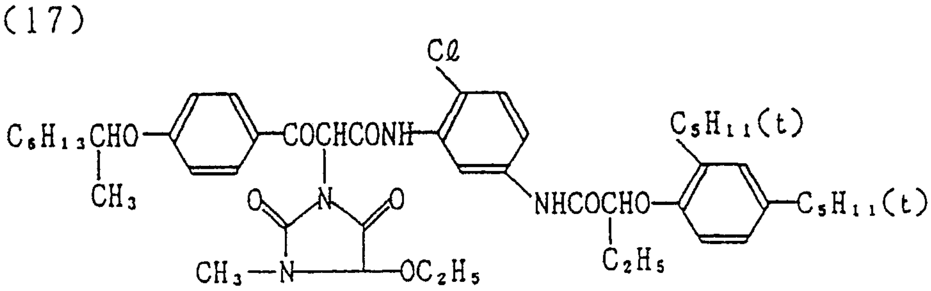

(18)

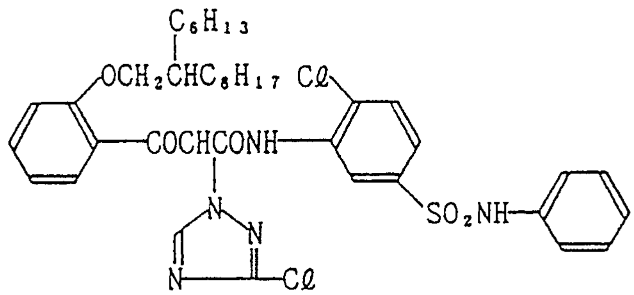

(19)

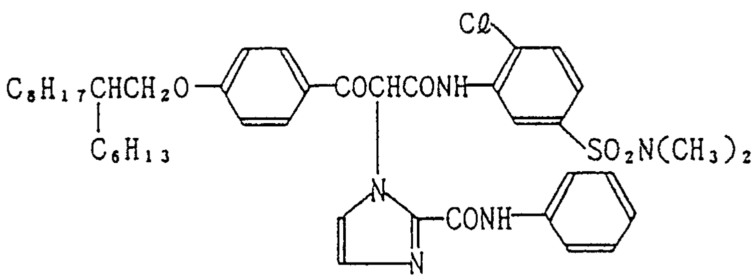

(20)

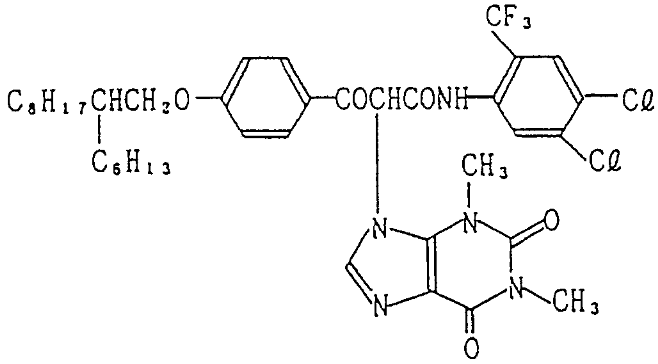

(21)

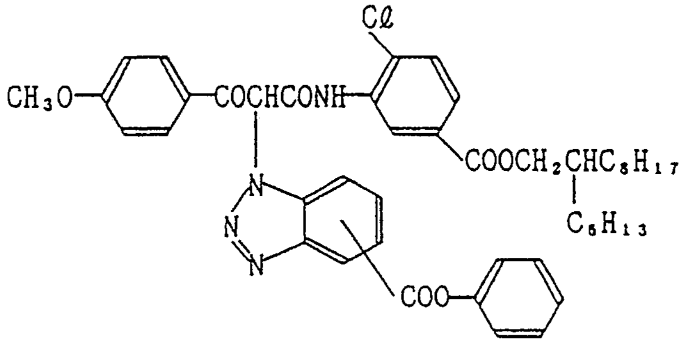

(22)

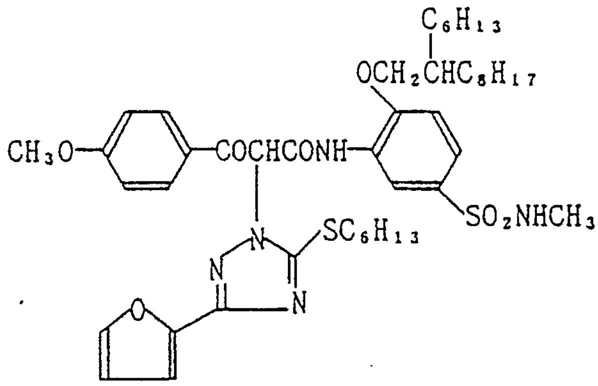

(23)

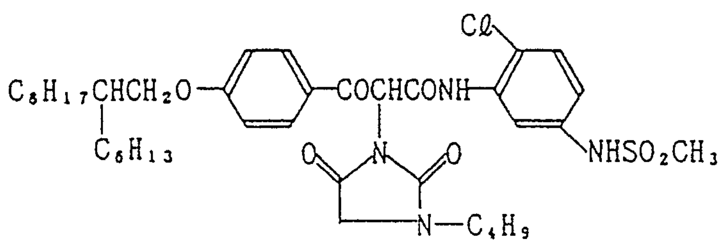

(24)

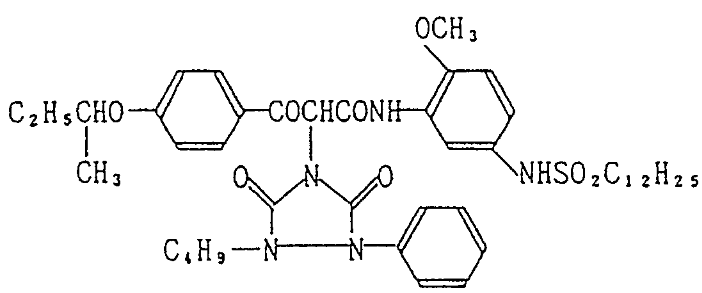

(25)

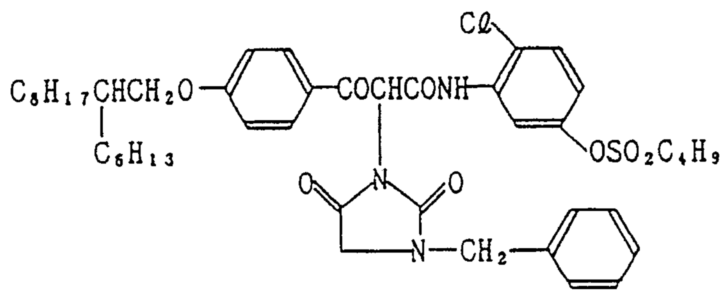

(26)

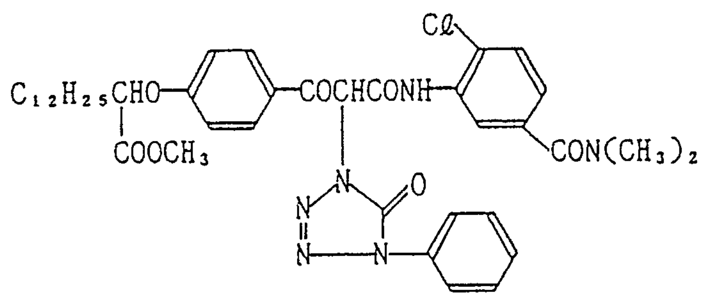

(27)

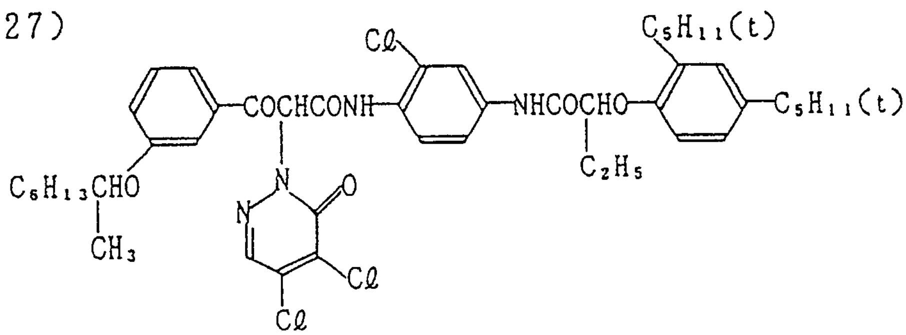

(28)

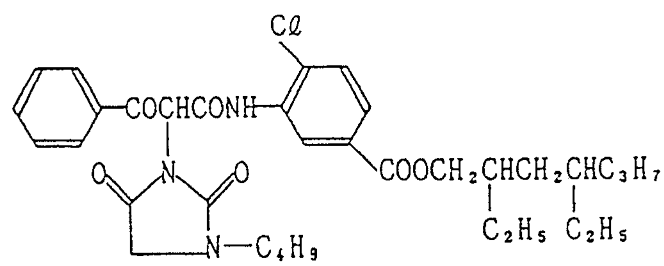

Next, the typical synthesis examples of the yellow couplers of the invention will be given below.

Synthesis Examples: Synthesis of Coupler 2

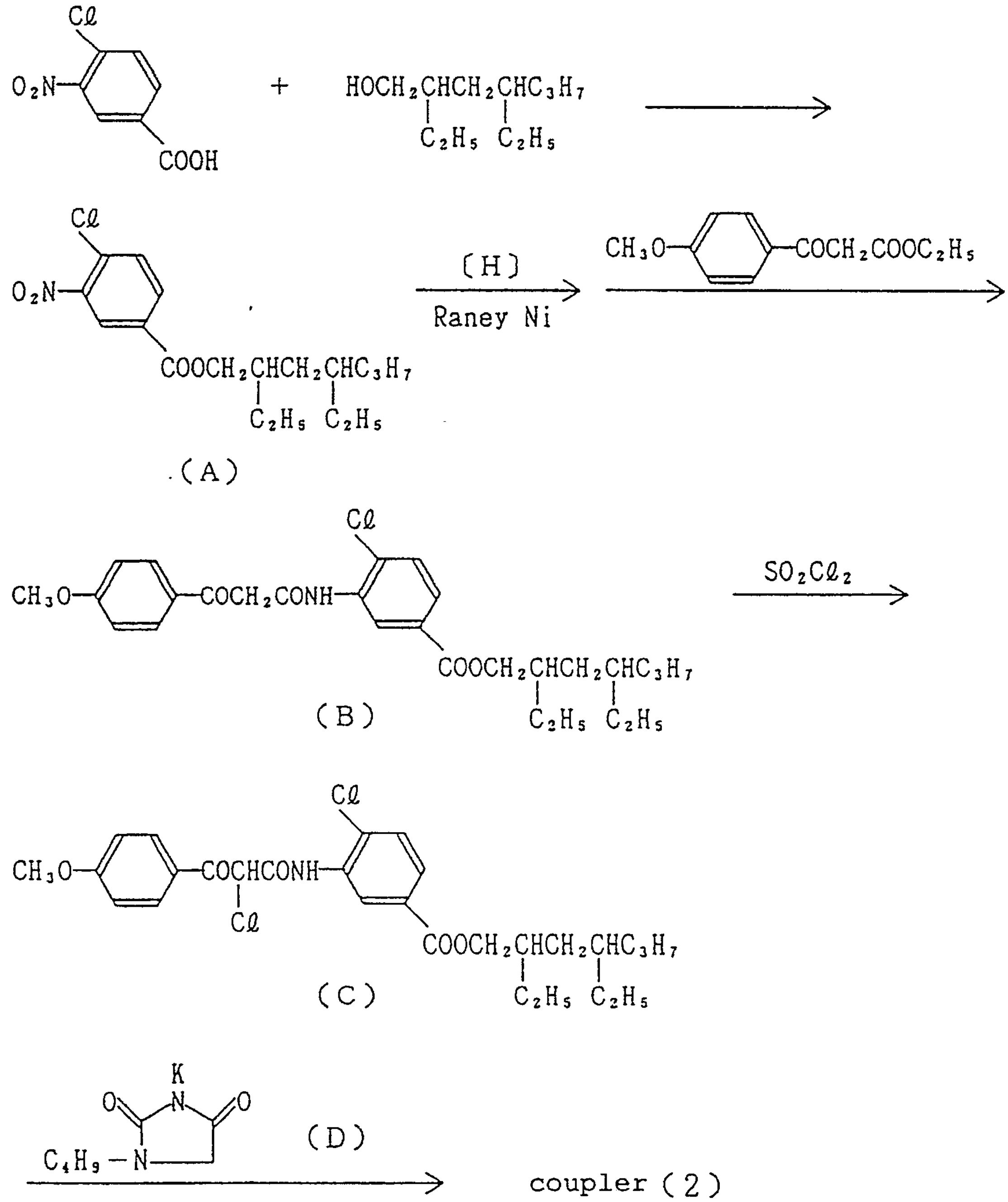


(A)

(B)

(C)

coupler (2)

4-chloro-3-nitrobenzoic acid of 40.3 g, 34.4 g of 2,4-diethylheptane-1-ol and 0.3 g of p-toluenesulfonic acid monohydrate were added into 500 mℓ of toluene and the mixture was refluxed with heating for 20 hours, while removing produced water by using Dean & Stark apparatus. The resulting reaction solution was rinsed with 200 mℓ of an aqueous 1N solution of sodium hydroxide and was then washed with water. A toluene layer was condensed under a reduced pressure, thereby obtaining 65.5 g of A.

Next, 60 g of A was dissolved in 300 mℓ of ethanol and 5 g of Raney nickel and 0.1 g of dicyan diamide were added thereto, so that a contact hydrogenation was carried out under atmospheric pressure. After absorbing hydrogen in a theoretical quantity, the Raney nickel was filtrated away and the ethanol solution was then condensed under reduced pressure. The condensed ethanol solution was added with 37.5 g of 3-(4-methoxyphenyl)-3-oxopropionic acid ethyl ester and 500 mℓ of xylene, so that the mixture was reacted for 5 hours while keeping the ethanol distilled off. After cooling it down to room temperature, 1000 mℓ of n-hexane was added thereto and the produced solids were filtrated, so that 43.2 g of B was obtained.

Then, 30 mℓ of chloroform was added to 5.0 g of B and the mixture was cooled with ice. After dropping 1.4 g of sulfuryl chloride thereinto, an agitation was made for one hour under cooling with ice and then for

two hours at room temperature. The resulting reaction solution was condensed under reduced pressure, so that C was obtained.

After adding 3.9 g of D and 100 mℓ of dimethyl formamide into the resulting C, the mixture thereof was reacted at a temperature of 70° C for two hours. The resulting reaction solution was added with 200 mℓ of ethyl acetate and 300 mℓ of water and was then extracted. After washing the ethyl acetate layer with water and then condensing it under reduced pressure, the residues were refined in silica gel chromatography, by making use of a mixed solvent comprising ethyl acetate and n-hexane in a proportion of 1 : 2 as the developing solvent. The resulting refined residues were recrystallized with methyl alcohol, so that 2.8 g of Coupler 2 was obtained.

The structure of Coupler 2 was confirmed by making use of NMR, IR and Mass spectrometry.

The other exemplified couplers of the invention were also synthesized by making use of the corresponding raw materials as the starting materials and then by following the synthesis examples.

The yellow couplers of the invention are allowed to use independently or in combination. It is further allowed to use them with any known pivaloyl acetoanilide type or a benzoyl acetoanilide type yellow couplers in combination.

A yellow coupler of the invention can be contained in the silver halide emulsion of a light-sensitive material in the following manner. The yellow coupler is dissolved in an independent high boiling solvent having a boiling point of not lower than 175° C, such as tricresyl phosphate and dibutyl phthalate, or an independent low boiling solvent such as ethyl acetate and butyl propionate, or the mixed solvent thereof, independently or in combination and, then, the solution is mixed up with an aqueous gelatin solution containing a surfactant. Next, the mixture is so emulsified as to be dispersed with a high-speed rotary mixer or a colloid mill and, then, the dispersion is added directly into a silver halide emulsion, the resulting emulsion is coated on a support and dried; or, after setting the emulsified dispersion and cutting it into minute pieces, the low boiling solvent is removed therefrom by means of washing treatment, for example, and the resulting matter is added into an emulsion, so that the resulting emulsion may be coated on a support and dried.

Generally, it is preferable to add the yellow coupler of the invention in an amount within the range of 10 ~ 300 g per mol of silver halide used, provided, the amount added may be varied in accordance with the purposes of application.

The silver halide photographic light-sensitive materials of the invention may be of any types, regardless of any kind and application. The silver halides applicable thereto include, for example, silver chloride, silver bromide, silver iodide, silver chlorobromide, silver iodobromide and silver chloroiodobromide.

In order to form a multicolor image, other color couplers as well as the yellow coupler of the invention may be contained in the silver halide photographic light-sensitive materials relating to the invention.

The silver halide photographic light-sensitive materials of the invention are allowed to freely contain an anti-stain agent, an image stabilizer, a hardener, a plasticizer, a polymer latex, a formalin scavenger, a mordant, a development accelerator, a development retarder, a fluorescent whitening agent, a matting agent, a solvent, an antistatic agent and a surfactant.

In addition, when a UV absorbent is contained in a silver halide photographic light-sensitive material containing the yellow coupler of the invention, the durability of a yellow image can further be improved.

## EXAMPLES

Next, the examples of the invention will be given below so that the invention may be further detailed. It is, however, to be understood that the embodiments of the invention shall not be limited thereto.

### Example 1

As shown in Table 1, the yellow couplers of the invention, which will be indicate correspondingly by the foregoing exemplified coupler numbers, and the following comparative couplers, each in an amount of 10.0 g, were added into a mixed solution of 2.0 mℓ of dibutyl phthalate and 20 mℓ of ethyl acetate, and the resulting mixture was dissolved by heating at 50° C.

The resulting solution was mixed up with both of 5 mℓ of an aqueous 10% solution of Alkanol B, alkylnaphthalene sulfonate, manufactured by DuPont, and 100 mℓ of an aqueous 5% gelatin solution, and

the mixture was emulsified by passing through a colloid mill several times repeatedly. After completing the emulsification, it was stored at 20 ° C for 48 hours and was then observed with eye through a 20X magnifier.

The results thereof are shown in Table 1.

The above-mentioned comparative couplers used therein were as follows:

Y-1, a coupler given in Japanese Patent Publication Open to Public Inspection (hereinafter referred to as Japanese Patent 0.P.I. Pubication) No. 50-34232/1975

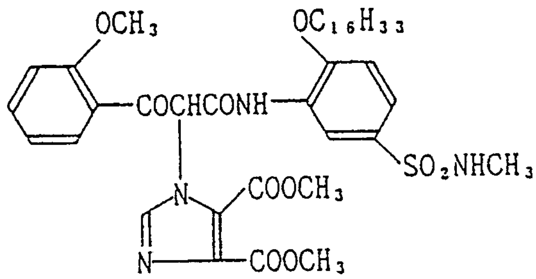

Y-2, a coupler given in Japanese Patent O.P.I. Publication No. 51-102636/1976

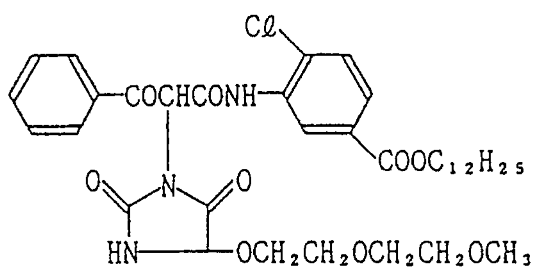

Y-3, a coupler given in Japanese Patent O.P.I. Publication No. 55-142340/1980

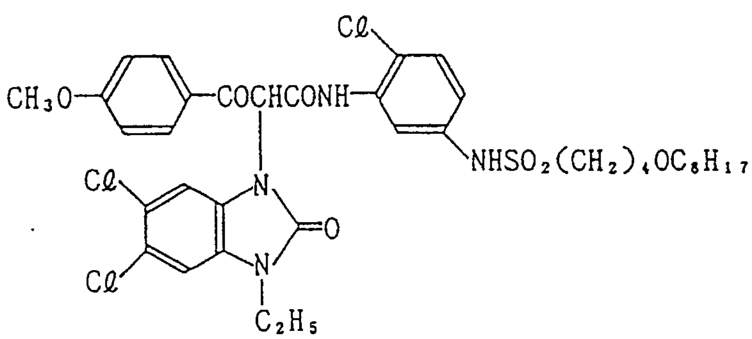

Y-4, a coupler given in Japanese Patent O.P.I. Publication No. 59-174839/1984

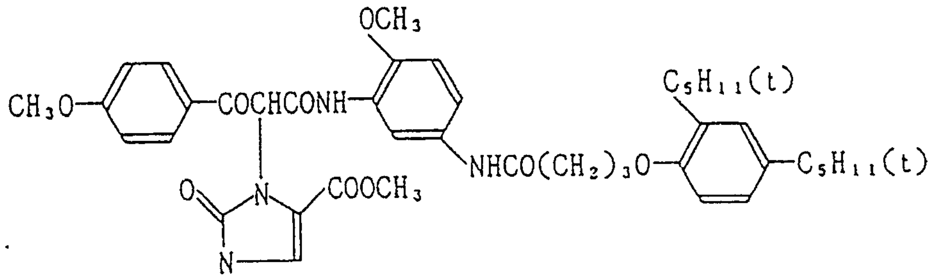

Y-5

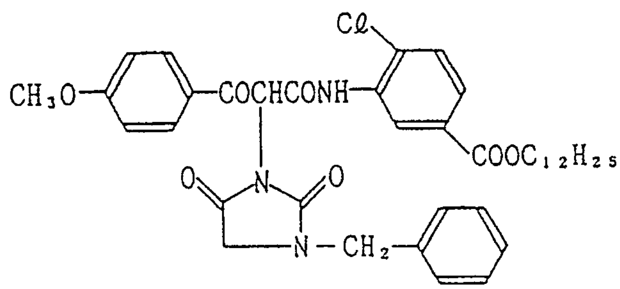

Table 1

| Sample No. | Coupler No. | Post-storage condition |
|---|---|---|
| 1, Invention | 4 | No deposition found |
| 2, Invention | 6 | No deposition found |
| 3, Invention | 10 | No deposition found |
| 4, Invention | 12 | No deposition found |
| 5, Invention | 15 | No deposition found |
| 6, Invention | 19 | No deposition found |
| 7, Invention | 24 | No deposition found |
| 8, Invention | 26 | No deposition found |
| 9, Invention | 27 | No deposition found |
| 10, Comparison | Y-1 | A few depositions found |
| 11, Comparison | Y-2 | Serious depositions found |
| 12, Comparison | Y-3 | Serious depositions found |
| 13, Comparison | Y-4 | A few depositions found |
| 14, Comparison | Y-5 | A few depositions found |

As is apparent from the above Table 1, it was proved that every yellow coupler of the invention had nothing deposited and an excellent dispersion stability when allowing them to stand, as compared to the comparative couplers. The effects of the invention displayed on the samples in which Coupler No. 28 was used in place of Coupler 4 of Sample No. 1.

Example 2

As shown in Table 2, 0.1 mols each of the yellow couplers of the invention and the following comparative couplers were weighed out per mol of silver, and dioctyl phthalater, as a high boiling solvent, in an amount by weight equivalent to that of the coupler used and ethyl acetate in an amount three times as much as that of the coupler used were each added thereinto and were then completely dissolved by heating up to a temperature of 60°C. The resulting solution was mixed up with 1200 mℓ of an aqueous 5% gelatin solution containing 120 mℓ of an aqueous 5% solution of Alkanol B, alkyl naphthalene sulfonate, manufactured by DuPont. The mixture was so dispersed as to obtain an emulsified dispersion by making use of a ultrasonic homogenizer. Next, this dispersion was added into 4 kg of a blue-sensitive silver iodobromide emulsion having a silver iodide content of 7 mol% and thereto 120 mℓ of a 2% 1,2-bis-(vinylsulfonyl)ethane solution having a proportion of water to methanol = 1 : 1 was added. The resulting emulsion was coated on a subbed transparent polyester film base so that the amount of silver iodobromide coated could be 15 mg/100 $dm^2$, and dried up, thereby preparing Samples No. 15 ~ No. 25 each of the silver halide photographic light-sensitive materials having the stable coated layers, respectively.

The resulting samples were exposed to white light through a wedge and were then color developed in the following color processing steps.

| Processing step (Carried out at 38°C) | |
|---|---|
| Color developing | 3min.15sec. |
| Bleaching | 6min.30sec. |
| Washing | 3min.15sec. |
| Fixing | 6min.30sec. |
| Washing | 3min.15sec. |
| Stabilizing | 1min.30sec. |
| Drying | |

The compositions of the processing solutions each used in the above processing steps were as follows:

| Color Developer A | |
|---|---|
| 4-amino-3-methyl-N-ethyl-N-(β-hydroxyethyl)aniline • sulfate | 4.75 g |
| Anhydrous sodium sulfite | 4.25 g |
| Hydroxylamine • 1/2 sulfate | 2.0 g |
| Anhydrous potassium carbonate | 37.5 g |
| Sodium bromide | 1.3 g |
| Trisodium nitrilotriacetate, monohydrate | 2.5 g |
| Potassium hydroxide | 1.0 g |
| Add water to make | 1 ℓ |
| Adjust pH with potassium hydroxide to be | pH 10.0 |

| Bleacher | |
|---|---|
| Iron-ammonium ethylenediaminetetraacetate | 100.0 g |
| Diammonium ethylenediaminetetraacetate | 10.0 g |
| Ammonium bromide | 150.0 g |
| Glacial acetic acid | 10.0 mℓ |
| Add water to make | 1 ℓ |
| Adjust pH with aqueous ammonia to be | pH 6.0 |

| Fixer | |
|---|---|
| Ammonium thiosulfate | 175.0 g |
| Anhydrous sodium sulfite | 8.5 g |
| Sodium metasulfite | 2.3 g |
| Add water to make | 1 ℓ |
| Adjust pH with acetic acid to be | pH 6.0 |

| Stabilizer | |
|---|---|
| Formalin in an queous 37% solution | 1.5 mℓ |
| Wetting agent, Koniducks, manufactured by Konica Corporation | 7.5 mℓ |
| Add water to make | 1 ℓ |

The same processing treatments were carried out with Color Developer B having the same composition with that of the above-described Color Developer A, except that pH was changed to be 9.8.

The density of each processed sample was measured through a blue filter and the relative values of the fog, maximum density and sensitivity, that is, a reciprocal number of an exposure capable of producing the density of fog + 0.1, are each shown in Table 2.

Table 2

| Sample No. | Coupler No. | Color developer A | | | Color developer B | | |
|---|---|---|---|---|---|---|---|
| | | Sensitivity* | Fog | Max. density | Sensitivity* | Fog | Max. density |
| 15, Invention | (1) | 100 | 0.02 | 2.82 | 92 | 0.02 | 2.61 |
| 16, Invention | (2) | 101 | 0.02 | 2.83 | 93 | 0.02 | 2.62 |
| 17, Invention | (5) | 114 | 0.03 | 2.97 | 102 | 0.02 | 2.78 |
| 18, Invention | (7) | 102 | 0.04 | 2.85 | 93 | 0.03 | 2.63 |
| 19, Invention | (13) | 103 | 0.03 | 2.85 | 93 | 0.02 | 2.62 |
| 20, Invention | (16) | 100 | 0.03 | 2.81 | 90 | 0.03 | 2.57 |
| 21, Invention | (20) | 97 | 0.04 | 2.76 | 90 | 0.03 | 2.58 |
| 22, Invention | (25) | 108 | 0.04 | 2.90 | 95 | 0.03 | 2.68 |
| 23, Comparison | Y-6 | 103 | 0.05 | 2.86 | 79 | 0.04 | 2.38 |
| 24, Comparison | Y-7 | 104 | 0.04 | 2.87 | 81 | 0.04 | 2.43 |
| 25, Comparison | Y-8 | 96 | 0.05 | 2.75 | 75 | 0.03 | 2.32 |

* Each ensitivity is expressed by a value relative to the sensitivity obtained when Sample No. 15 was processed with Color developer A, which is regarded as a value of 100.

Y-6, a coupler given in European Patent No. 327,348

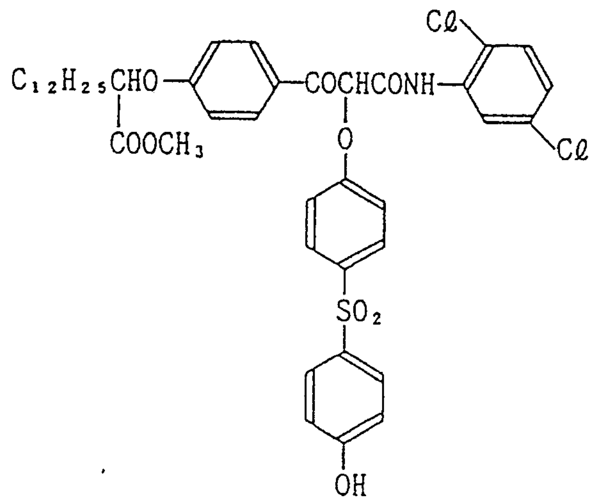

Y-7

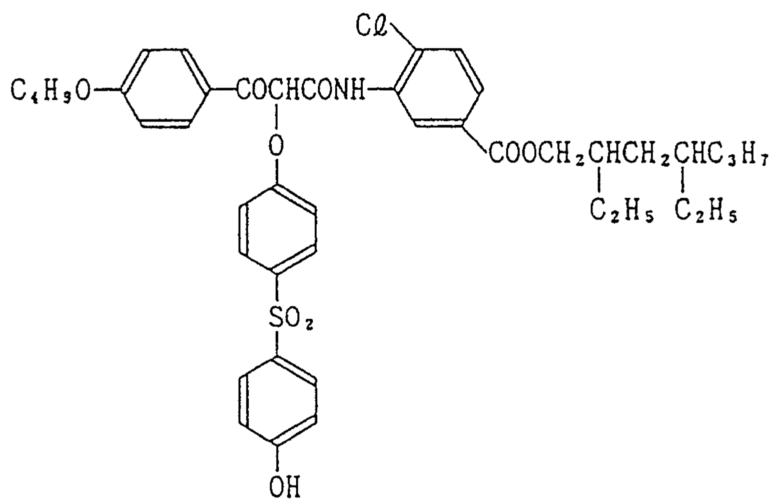

Y-8

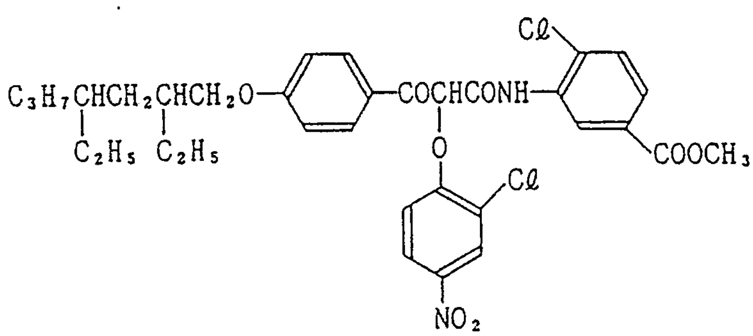

As is obvious from Table 2, as compared to the comparative samples, the samples of the invention are excellent in sensitivity and maximum density, i.e.,color developability, even in the case where the pH of a color developer is lowered.

Example 3

The samples of the silver halide color photographic light-sensitive materials were prepared in the same manner as in Example 2, except that yellow coupler 2 of the invention and comparative coupler Y-3 were used and the amount of dioctyl phthalate was changed as shown in Table 3. The resulting samples were each processed in the same treatments with color developer A and the maximum densities thereof were measured.

As shown in Table 3, the results thereof proved that the couplers of the invention are excellent in solubility and dispersion stability and capable of displaying a stable color developability without seriously affecting the photographic characteristics even when changing an amount of a high boiling solvent used.

Table 3

| Sample No. | Coupler | DOP/Coupler | |
|---|---|---|---|
| 26 | Exemplified coupler 2 | 1/2 | 2.82 |
| 27 | -do- | 1/4 | 2.81 |
| 28 | -do- | 1/8 | 2.79 |
| 29 | Comparative coupler Y-3 | 1/2 | 2.80 |
| 30 | -do- | 1/4 | 2.51 |
| 31 | -do- | 1/8 | 2.26 |

Example 4

Multilayered color photographic element sample No. 32 was prepared coating each of the layers having the following compositions on a triacetyl cellulose film support, in order from the support side.

Layer 1 : An antihalation layer, HC-1, that was a gelatin layer containing black colloidal silver

Layer 2 : An interlayer, I.L., that was a gelatin layer

Layer 3 : A low-speed red-sensitive emulsion layer, RL-1, that was a layer containing the following components:

Monodisperse emulsion, Em-I, having an average grain-size $\bar{r}$ of 0.30 µm and comprising AgBrI having an AgI content of 6 mol%. •••••Amount of silver coated: 1.8 g/m$^2$

Sensitizing dye I••••$6x10^{-5}$ mols per mol of silver

Sensitizing dye II•••$1.0x10^{-5}$ mols per mol of silver

Cyan coupler, C-2••••0.06 mols per mol of silver

Colored cyan coupler, CC-1 •••••0.003 mols per mol of silver

DIR compound, D-1••••0.0015 mols per mol of silver

DIR compound, D-2••••0.002 mols per mol of silver

Tricresyl phosphate••0.3 mℓ/m$^2$

Layer 4 : A high-speed red-sensitive emulsion layer, RH-1, that was a layer containing the following components:

Monodisperse emulsion, Em-II, having an average grain-size $\bar{r}$ of 0.5 µm and comprising AgBrI having an AgI content of 7.0 mol% •••••Amount of silver coated: 1.3 g/m$^2$

Sensitizing dye I••••$3x10^{-5}$ mols per mol of silver

Sensitizing dye II•••$1.0x10^{-5}$ mols per mol of silver

Cyan coupler, C-2••••0.02 mols per mol of silver

Colored cyan coupler, CC-1 •••••0.0015 mols per mol of silver

DIR compound, D-2••••0.001 mols per mol of silver

Tricresyl phosphate•••••••••0.1 mℓ/m$^2$

Layer 5 : An interlayer, I.L., that was the same gelatin layer as Layer 2

Layer 6 : A low-speed green-sensitive emulsion layer, GL-1, containing the following components:

Em-1•••••••••Amount of silver coated: 1.5 g/m$^2$

Sensitizing dye III••2.5x$10^{-5}$ mols per mol of silver
Sensitizing dye IV•••1.2x$10^{-5}$ mols per mol of silver
Magenta coupler, M-2•••0.050 mols per mol of silver
Colored magenta coupler, CM-1 •••••0.009 mols per mol of silver
DIR compound, D-1••••••0.0010 mols per mol of silver
DIR compound, D-3••••••0.0030 mols per mol of silver
Tricresyl phosphate••••1.4 mℓ/$m^2$
Layer 7 : A high-speed green-sensitive emulsion layer, GH-1, containing the following components:
Em-II••••••••Amount of silver coated: 1.4 g/$m^2$
Sensitizing dye III••1.5x$10^{-5}$ mols per mol of silver
Sensitizing dye IV•••1.0x$10^{-5}$ mols per mol of silver
Magenta coupler, M-2•••0.020 mols per mol of silver
Colored magenta coupler, CM-1 •••••0.002 mols per mol of silver
DIR compound, D-3••••••0.0010 mols per mol of silver
Tricresyl phosphate••••0.8 mℓ/$m^2$
Layer 8 : A yellow filter layer, YC-1, that was a gelatin layer containing yellow colloidal silver
Layer 9 : A low-speed blue-sensitive emulsion layer, BL-1, containing the following components:
Monodisperse emulsion, Em-III, having an average grain-size of 0.48 µm and comprising AgBrI having an AgI content of 6 mol% •••••Amount of silver coated: 0.9 g/$m^2$
Sensitizing dye V••••1.3x$10^{-5}$ mols per mol of silver
Comparative coupler, Y-1 •••••0.29 mols per mol of silver
Tricresyl phosphate•••••••0.7 mℓ/$m^2$
Layer 10: A high-speed blue-sensitive emulsion layer, BH-1, having the following components:
Monodisperse emulsion, Em-IV, having an average grain-size of 0.8 µm and comprising AgBrI having an AgI content of 15 mol% •••••Amount of silver coated: 0.5 g/$m^2$
Sensitizing dye V••••1.0x$10^{-5}$ mols per mol of silver
Comparative coupler, Y-1 •••••0.08 mols per mol of silver
DIR compound, D-2••••••0.0015 mols per mol of silver
Tricresyl phosphate•••••••0.2 mℓ/$m^2$
Layer 11: The 1st protective layer, Pro-1, that was a gelatin layer containing silver iodobromide having an AgI content of 1 mol%, an average grain-size of 0.07 µm and the amount coated of 0.5 g/$m^2$, UV absorbents of UV-3 of 0.1 g/$m^2$ and UV-4 of 0.05 g/$m^2$ and tricresyl phosphate of 0.2 mℓ/$m^2$.
Layer 12: The 2nd protective layer, Pro-2, that was a gelatin layer containing polymethyl methacrylate particles having a diameter of 1.5 µm and formalin scavenger HS-1.

To each of the layers, gelatin hardener H-1 and a surfactant were added, beside the above-given compositions.

The compounds added into each of the layers of Sample No. 1 were as follows:
Sensitizing dye I : Anhydro-5,5′-dichloro-9-ethyl-3,3′-di-(3-sulfopropyl)thiacarbocyanine hydroxide
Sensitizing dye II : Anhydro-9-ethyl-3,3′-di-(3-sulfopropyl)-4,5,4′5′-dibenzothiacarbocyanine hydroxide
Sensitizing dye III : Anhydro-5,5′-diphenyl-9-ethyl-3,3′-di-(3-sulfopropyl)oxacarbocyanine hydroxide
Sensitizing dye IV : Anhydro-9-ethyl-3,3′-di-(3-sulfopropyl)-5,6,5′,6′-dibenzooxacarbocyanine hydroxide
Sensitizing dye V : Anhydro-3,3′-di-(3-sulfopropyl)-4,5-benzo-5′-methoxythiacyanine hydroxide

C － 2

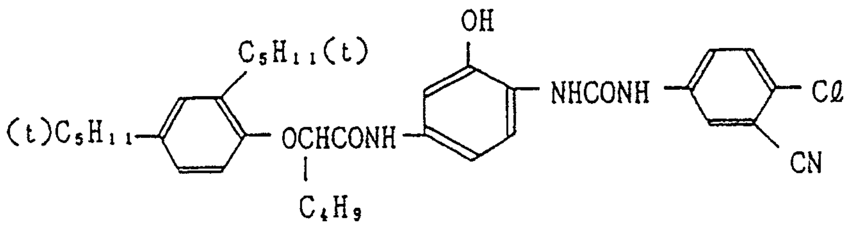

C C － 1

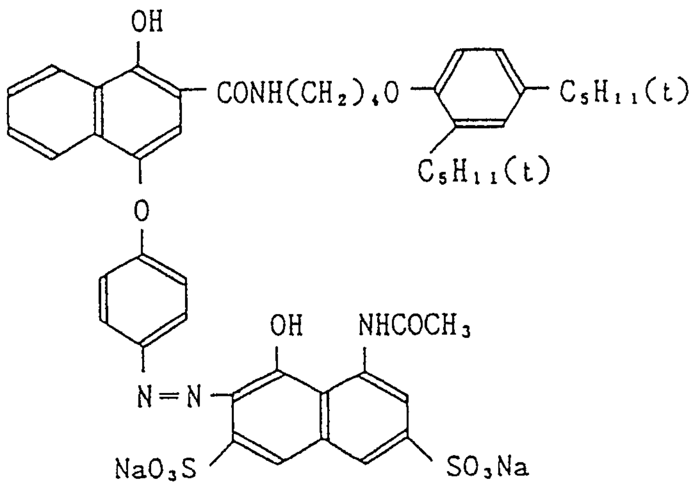

D － 1

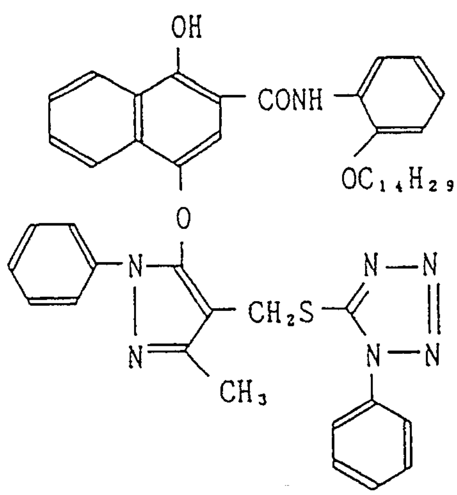

D - 2

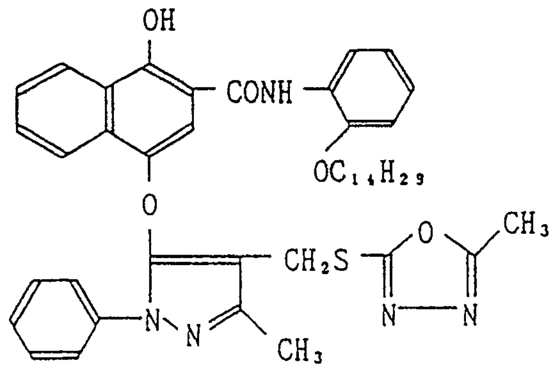

D - 3

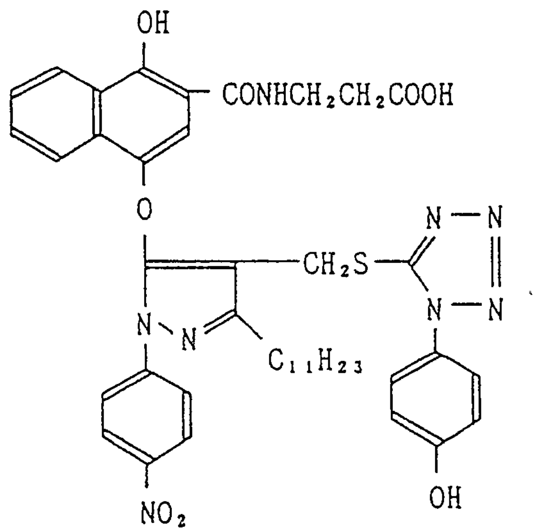

M - 2

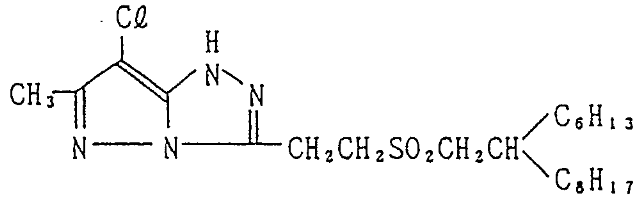

C M − 1

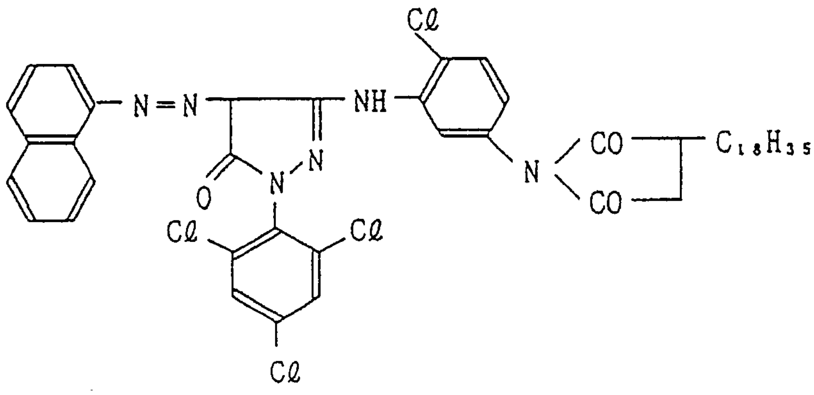

U V − 3

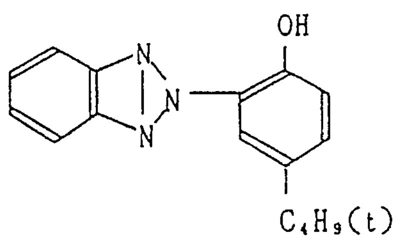

U V − 4

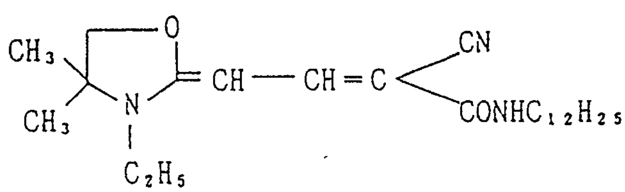

H S − 1

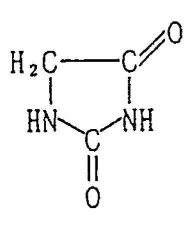

H − 1

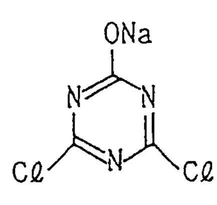

Further, Samples No. 33 through No. 37 were prepared by changing Comparative Coupler Y-1 used in Layers 9 and 10 of Sample No. 32 into those shown in Table 4.

In the preparations, the yellow densities were so adjusted as to be the same with each other, while the quantitative proportion of the couplers to tricresyl phosphate remained constant. The resulting samples were exposed to white light through a pattern for measuring MTF and were then processed in the same manner as in Example 3.

For evaluating the sharpness of each of the samples after completing the processing treatments, the MTF values thereof were measured in terms of a spatial frequency of 20 cycles/mm.

The results thereof are shown in Table 4.

Table 4

| Sample No. | Coupler No. | MTF value* |
|---|---|---|
| 32, Comparison | Y-1 | 100 |
| 33, Comparative | Y-4 | 98 |
| 34, Invention | 3 | 108 |
| 35, Invention | 8 | 106 |
| 36, Invention | 14 | 107 |
| 37, Invention | 23 | 107 |

* MTF values are indicated by a relative value to the MTF value of Sample No. 32 which is regarded as a value of 100.

As is apparent from Table 4, it was proved that the yellow couplers of the invention are effective for improving the sharpness, as compared to the comparative couplers.

## Claims

1. A silver halide color photographic light-sensitive material comprising a layer containing a coupler represented by the following Formula I :

Formula I

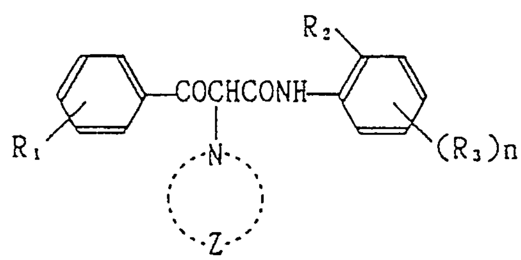

wherein $R_1$ represents a hydrogen atom, an alkyl group, an alkoxy group, or an acylamino group; $R_2$ represents a halogen atom, a trifluoromethyl group, or an alkoxy group; $R_3$ represents $L-R_4$ or a halogen atom, in which L represents -OCO-, -COO-, -O-, -S-, $-SO_2-$,

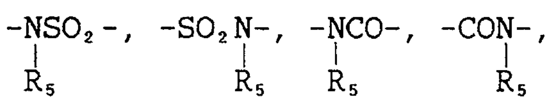

or $-OSO_2-$, $R_4$ represents an alkyl group or an aryl group, and $R_5$ represents a hydrogen atom or an alkyl group; n is an integer of 0, 1 or 2; and Z represents a group consisting of non-metal atoms necessary for forming a 5- or 6-membered heterocyclic ring; provided, when n is 2, $R_3$ may be the same with or different from each other; and at least one of $R_1$, $R_2$ and $R_3$ is a branched alkyl group having not less than 4 carbon atoms containing an asymmetric carbon atom, or a group containing said branched alkyl group.

2. A color photographic light-sensitive material according to claim 1, wherein the group represented by $R_1$ is a hydrogen atom, a straight-chained or branched alkyl group having from 1 to 30 carbon atoms, a straight-chained or branched alkoxy group having from 1 to 30 carbon atoms, or a straight-chained or branched alkylcarbonylamino or arylcarbonylamino group each having from 2 to 30 carbon atoms.

3. A color photographic light-sensitive material according to claim 2, wherein $R_1$ is an alkoxy group.

4. A color photographic light-sensitive material according to claim 1, wherein the group represented by $R_2$ is a halogen atom, a trifluoromehyl group, or a straight-chained or branched alkoxy group having from 1 to 30 carbon atoms.

5. A color photographic light-sensitive material according to claim 4, wherein $R_2$ is a chlorine atom.

6. A color photographic light-sensitive material according to claim 1, wherein the group represented by $R_4$ is a straight-chained or branched alkyl group having from 1 to 30 carbon atoms, a phenyl group, or a naphthyl group.

7. A color photographic light-sensitive material according to claim 6, wherein $R_4$ is a straight-chained or branched alkyl group having from 1 to 30 carbon atoms.

8. A color photographic light-sensitive material according to claim 1, wherein the group represented by L is -COO-,

$$-\underset{R_5}{N}SO_2-,\quad -SO_2\underset{R_5}{N}-\ \text{or}\ -OSO_2-.$$

9. A color photographic light-sensitive material according to claim 8, wherein L is -COO-.

10. A color photographic light-sensitive material according to claim 8, wherein the group represented by $R_5$ is a hydrogen atom, or a straight-chained or branched alkyl group having from 1 to 30 carbon atoms.

11. A color photographic light-sensitive material according to claim 1, wherein the 5- or 6-membered heterocyclic ring formed by Z is selected from the ring represented by the following formula II to VII :

II

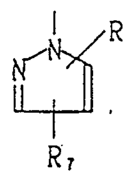

III

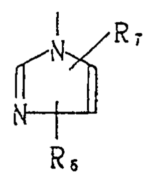

IV

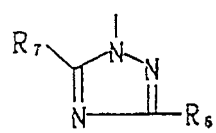

wherein $R_6$ and $R_7$ each represent a hydrogen atom, a halogen atom, an alkyl group, an alkoxy group, an aryl group, a heterocyclic group, an alkoxycarbonyl group, an aryloxycarbonyl group, an amino group, an acylamino group, an alkylsulfonyl group, an arylsulfonyl group, an alkylsulfinyl group, an arylsulfinyl group, an alkylsulfonamido group, an arylsulfonamido group or a carboxyl group, and $R_6$ and $R_7$ may form a ring;

V

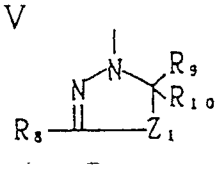

VI

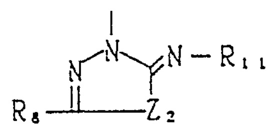

wherein $Z_1$ and $Z_2$ each represent an oxygen atom or a sulfur atom, and $R_8$, $R_9$ and $R_{10}$ represent each the same groups as those represented by $R_7$; and $R_{11}$ represents each an alkyl group, an aryl group, an alkylcarbonyl group, an arylcarbonyl group, an alkylsulfonyl group or an arylsulfonyl group, each of which may be further substituted;

VII

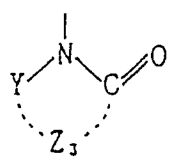

wherein Y represents -N=, -NH-, -O-, -S-, a sulfonyl group, a carbonyl group,

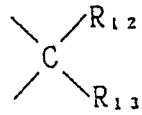

or

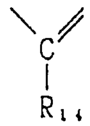

in which $R_{12}$, $R_{13}$, and $R_{14}$ represent each the same groups as those represented by $R_6$ and $R_7$, and may form a ring in association with a part of the rings formed by $Z_3$; and $Z_3$ represents a group consisting of non-metal atoms necessary for forming a 5- or 6-membered ring in association with -Y-N-CO-.

12. A color photographic light-sensitive material according to claim 1, wherein the material comprises a silver halide emulsion layer containing the coupler represented by Formula I in an amount of 10 to 300 g per mol of silver halide.